(19) Europäisches Patentamt
European Patent Office
Office européen des brevets

(11) **EP 4 643 771 A1**

(12) **EUROPEAN PATENT APPLICATION**

(43) Date of publication:
**05.11.2025 Bulletin 2025/45**

(21) Application number: **25173707.8**

(22) Date of filing: **30.04.2025**

(51) International Patent Classification (IPC):
**A61B 5/107** (2006.01)    **A61B 5/00** (2006.01)

(52) Cooperative Patent Classification (CPC):
**A61B 5/1076; A61B 5/4233; A61B 5/743;**
**A61B 5/7435;** A61B 2560/0487; G16H 30/40

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH LA MA MD TN**

(30) Priority: **01.05.2024   US 202463641132 P**
**01.05.2024   US 202463641126 P**
**29.04.2025   US 202519192973**

(71) Applicant: **Given Imaging Ltd.**
**20692 Yoqneam (IL)**

(72) Inventors:
• **NADIR, Lotem**
**Zichron Yaakov (IL)**
• **DEKEL, Eyal**
**Haifa (IL)**

(74) Representative: **HGF**
**HGF Limited**
**1 City Walk**
**Leeds LS11 9DX (GB)**

(54) **GRAPHICAL USER INTERFACE AND PROCESSING FOR ENDOLUMINAL FUNCTIONAL LUMEN IMAGING PROBE DATA**

(57)    A method includes: displaying a graphical user interface (GUI) including a 2D image representative of estimated diameters of an endoluminal tract; displaying, in the GUI, at least one user interface (UI) element among: a first UI element marking location of a esophago-gastric junction, a second UI element marking time and duration of a filling volume event, a third UI element marking time and duration of a dry catheter artifact event, and fourth UI elements marking pressure peak; determining usable data from the 2D image based on the at least one user interface element; computing, based on the usable data from the 2D image, at least one of: a distensibility index, or a maximum diameter of the EGJ; and displaying at least one of: the distensibility index, or the maximum diameter of the EGJ.

FIG. 1

**Description**

CROSS-REFERENCE TO RELATED APPLICATIONS

The present application claims the benefit of and priority to U.S. Provisional Application No. 63/641,126, filed May 1, 2024, and U.S. Provisional Application No. 63/641,132, filed May 1, 2024. Each of the foregoing applications is hereby incorporated by reference herein in its entirety.

FIELD

This disclosure relates to endoluminal functional lumen imaging probe (EndoFLIP) data, and more particularly, to a graphical user interface and processing for EndoFLIP data.

BACKGROUND

The esophagus is a tubular organ that carries food and liquid from the throat to the stomach. Accurate measurements of physiological parameters of the esophagus under realistic swallowing conditions are valuable in diagnosing esophageal diseases such as achalasia, dysphagia, diffuse esophageal spasm, ineffective esophageal motility, and hypertensive lower esophageal sphincter (LES). When a person with a healthy esophagus swallows, circular muscles in the esophagus contract. The contractions begin at the upper end of the esophagus and propagate downwardly toward the lower esophageal sphincter (LES). The function of the peristaltic muscle contractions, i.e., to propel food and drinks through the esophagus to the stomach, is sometimes called the motility function but is also often referred to as peristalsis. Esophageal manometry is a procedure used to assess pressure and motor function of the esophagus, allowing physicians to evaluate how well the muscles in the esophagus work to transport liquids or food from the mouth into the stomach.

SUMMARY

In accordance with aspects of the disclosure, a system includes at least one processor, and at least one memory storing instructions. The instructions, when executed by the at least one processor, cause the system to at least perform: displaying a graphical user interface (GUI) including a two-dimensional (2D) image representative of estimated diameters of an endoluminal tract, where the 2D image has a first dimension representative of endoluminal positions, a second dimension representative of points in time, and image pixels having pixel values representative of endoluminal diameter, where the endoluminal positions include a position of an esophago-gastric junction (EGJ); displaying, in the GUI, at least one user interface element among: a first user interface element marking location of the EGJ, a second user interface element marking time and duration of a filling volume event in which an endoluminal functional lumen imaging probe (EndoFLIP) is marked as being filled with liquid, a third user interface element marking time and duration of a dry catheter artifact (DCA) event, and a plurality of fourth user interface elements marking pressure peak points of pressure data captured by the EndoFLIP; determining usable data from the 2D image based on the at least one user interface element; computing, based on the usable data from the 2D image, at least one of: a distensibility index, or a maximum diameter of the EGJ; and displaying at least one of: the distensibility index, or the maximum diameter of the EGJ.

In accordance with aspects of the present disclosure, a processor-implemented method includes: displaying a graphical user interface (GUI) including a two-dimensional (2D) image representative of estimated diameters of an endoluminal tract, where the 2D image has a first dimension representative of endoluminal positions, a second dimension representative of points in time, and image pixels having pixel values representative of endoluminal diameter, where the endoluminal positions include a position of an esophago-gastric junction (EGJ); displaying, in the GUI, at least one user interface element among: a first user interface element marking location of the EGJ, a second user interface element marking time and duration of a filling volume event in which an endoluminal functional lumen imaging probe (EndoFLIP) is marked as being filled with liquid, a third user interface element marking time and duration of a dry catheter artifact (DCA) event, and a plurality of fourth user interface elements marking pressure peak points of pressure data captured by the EndoFLIP; determining usable data from the 2D image based on the at least one user interface element; computing, based on the usable data from the 2D image, at least one of: a distensibility index, or a maximum diameter of the EGJ; and displaying at least one of: the distensibility index, or the maximum diameter of the EGJ.

In accordance with aspects of the present disclosure, a non-transitory processor-readable medium stores instructions which, when executed by at least one processor of a system, cause the system to at least perform: displaying a graphical user interface (GUI) including a two-dimensional (2D) image representative of estimated diameters of an endoluminal tract, where the 2D image has a first dimension representative of endoluminal positions, a second dimension representative of points in time, and image pixels having pixel values representative of endoluminal diameter, where the endoluminal positions include a position of an esophago-gastric junction (EGJ); displaying, in the GUI, at least one user interface element among: a first user interface element marking location of the EGJ, a second user interface element marking time and duration of a filling volume event in which an endoluminal functional lumen imaging probe (EndoFLIP) is marked as being filled with liquid, a third user interface element marking time and duration of a dry catheter artifact (DCA) event, and a plurality of fourth user interface elements marking pressure peak points of pressure data captured by the EndoFLIP; determining usable data from the 2D image based on the at least one user interface element; computing, based on the usable data from the 2D image, at least one of: a distensibility index, or a maximum diameter of the EGJ; and displaying at least one of: the distensibility index, or the maximum diameter of the EGJ.

In accordance with aspects of the disclosure, a system includes at least one processor, and at least one memory storing instructions. The instructions, when executed by the at least one processor, causes the system to at least perform: accessing a two-dimensional (2D) image representative of estimated diameters of an endoluminal tract, where the 2D image has a first dimension representative of endoluminal positions, a second dimension representative of points in time, and image pixels having pixel values representative of endoluminal diameter, where the endoluminal positions include a position of an esophago-gastric junction; determining contours around a pixel subset of the image pixels, where the pixel subset contains pixels which represent endoluminal diameters that exceed a threshold diameter; for the contours, identifying a lowest contour point of each time point of the contours, to provide lowest contour points; determining local minimum points of the lowest contour points; estimating a location of the esophago-gastric junction based on the local minimum points; and displaying the estimated location of the esophago-gastric junction relative to the 2D image.

In accordance with aspects of the present disclosure, a processor-implemented method includes: accessing a two-dimensional (2D) image representative of estimated diameters of an endoluminal tract, where the 2D image has a first dimension representative of endoluminal positions, a second dimension representative of points in time, and image pixels having pixel values representative of endoluminal diameter, where the endoluminal positions include a position of an esophago-gastric junction; determining contours around a pixel subset of the image pixels, where the pixel subset contains pixels which represent endoluminal diameters that exceed a threshold diameter; for the contours, identifying a lowest contour point of each time point of the contours, to provide lowest contour points; determining local minimum points of the lowest contour points; estimating a location of the esophago-gastric junction based on the local minimum points; and displaying the estimated location of the esophago-gastric junction relative to the 2D image.

In accordance with aspects of the present disclosure, a non-transitory processor-readable medium stores instructions which, when executed by at least one processor of a system, cause the system to at least perform: accessing a two-dimensional (2D) image representative of estimated diameters of an endoluminal tract, where the 2D image has a first dimension representative of endoluminal positions, a second dimension representative of points in time, and image pixels having pixel values representative of endoluminal diameter, where the endoluminal positions include a position of an esophago-gastric junction; determining contours around a pixel subset of the image pixels, where the pixel subset contains pixels which represent endoluminal diameters that exceed a threshold diameter; for the contours, identifying a lowest contour point of each time point of the contours, to provide lowest contour points; determining local minimum points of the lowest contour points; estimating a location of the esophago-gastric junction based on the local minimum points; and displaying the estimated location of the esophago-gastric junction relative to the 2D image.

The details of one or more embodiments of the disclosure are set forth in the accompanying drawings and the description below. Other features, objects, and advantages of the techniques described in this disclosure will be apparent from the description and drawings, and from the claims.

BRIEF DESCRIPTION OF DRAWINGS

A detailed description of embodiments of the disclosure will be made with reference to the accompanying drawings, wherein like numerals designate corresponding parts in the figures:

FIG. 1 is a diagram of an example endoluminal functional lumen imaging probe (EndoFLIP) system, in accordance with aspects of the disclosure;
FIG. 2 is a block diagram of an example controller configured for use with the EndoFLIP system of FIG. 1, in accordance with aspects of the present disclosure;
FIG. 3 is a diagram of an example graphical user interface, in accordance with aspects of the disclosure;
FIG. 4 is an example two-dimensional (2D) image having a first dimension representative of endoluminal positions, a second dimension representative of points in time, and image pixels having pixel values indicative of endoluminal diameter, in accordance with aspects of the disclosure;
FIG. 5 is an example of the 2D image of FIG. 4 with a subset of pixels converted to black pixels, in accordance with aspects of the disclosure;
FIG. 6 is an example of the 2D image of FIG. 5 with another subset of pixels converted to white pixels, in accordance with aspects of the disclosure;

FIG. 7 is an example of the 2D image of FIG. 6 with illustrated contours of the black pixels, in accordance with aspects of the disclosure;

FIG. 8 is an example of the 2D image of FIG. 7 in which contour points for a single point of time are determined, in accordance with aspects of the disclosure;

FIG. 9 is an example of the 2D image of FIG. 8 in which the lowest contour point for a single point of time is identified, in accordance with aspects of the disclosure;

FIG. 10 is an example of the 2D image of FIG. 9 in which the lowest contour point for every point of time is shown, in accordance with aspects of the disclosure;

FIG. 11 is an example of the 2D image of FIG. 10 in which local minimum points are detected among the lowest contour points, in accordance with aspects of the disclosure;

FIG. 12 is an example of the 2D image of FIG. 4 in which an estimated lower boundary of the esophago-gastric junction is shown, in accordance with aspects of the disclosure;

FIG. 13 is an example of the 2D image of FIG. 12 in which an estimated location of the esophago-gastric junction is illustrated, in accordance with aspects of the disclosure;

FIG. 14 is a flow diagram of an example operation, in accordance with aspects of the disclosure;

FIG. 15 is a diagram of an example graphical user interface (GUI) for EndoFLIP data that includes user interface elements that can be added, moved, adjusted, and/or removed by a user, in accordance with aspects of the disclosure;

FIG. 16 is a diagram of the GUI of FIG. 15 in which a GUI element indicating a marked location of an esophago-gastric junction is adjusted by a user pixel, in accordance with aspects of the disclosure;

FIG. 17 is a diagram of the GUI of FIG. 15 in which a GUI element indicating a marked duration of dry catheter (DCA) is adjusted by a user, in accordance with aspects of the disclosure;

FIG. 18 is a diagram of the GUI of FIG. 15 in which GUI elements indicating marked pressure points adjusted by a user, in accordance with aspects of the disclosure; and

FIG. 19 is a flow diagram of an example operation, in accordance with aspects of the disclosure.

DETAILED DESCRIPTION

The disclosed technology will now be described in detail with reference to the drawings in which like reference numerals designate identical or corresponding elements in each of the several views. However, it is to be understood that the aspects of the disclosure are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure.

In the following description, certain specific details are set forth in order to provide a thorough understanding of disclosed aspects. However, one skilled in the relevant art will recognize that aspects may be practiced without one or more of these specific details or with other methods, components, materials, etc. In other instances, well-known structures associated with transmitters, receivers, or transceivers have not been shown or described in detail to avoid unnecessarily obscuring descriptions of the aspects.

Reference throughout this specification to "one aspect" or "an aspect" means that a particular feature, structure, or characteristic described in connection with the aspect is included in at least one aspect. Thus, the appearances of the phrases "in one aspect" or "in an aspect" in various places throughout this specification are not necessarily all referring to the same aspect. Furthermore, the particular features, structures, or characteristics may be combined in any suitable manner in one or more aspects.

An endoluminal functional lumen imaging probe (EndoFLIP) is a system used to assess pressure and motor function of the esophagus, allowing physicians to evaluate how well the muscles in the esophagus work to transport liquids or food from the mouth into the stomach. To perform this procedure, the EndoFLIP system operates in conjunction with a catheter probe placed in the esophagus of a patient to record pressure and impedance data over a period of time using various sensors placed on the catheter. The data is analyzed using analysis software to evaluate causes of and help diagnose conditions such as gastric reflux, difficulty swallowing, functional chest pain, achalasia, and hiatal hernia.

The disclosed EndoFLIP system obtains high resolution and/or three-dimensional (3D) mapping of endoluminal diameters within the tubular organs of the human gastrointestinal tract based on pressure with impedance levels within the tubular organs of the human upper gastrointestinal tract. The EndoFLIP system is used in a medical clinical setting to acquire the pressure and impedance levels and store the corresponding data for visualization and analysis using the software.

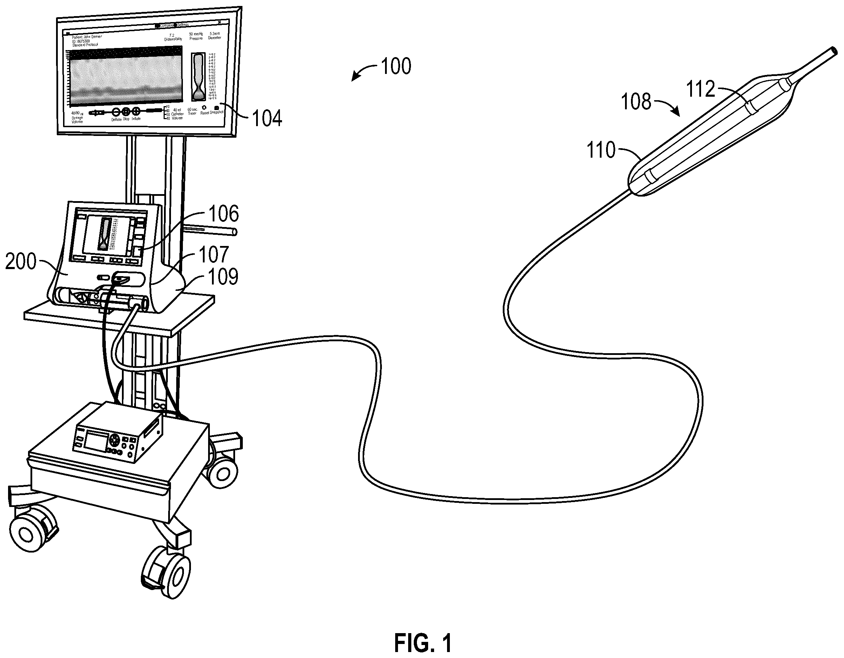

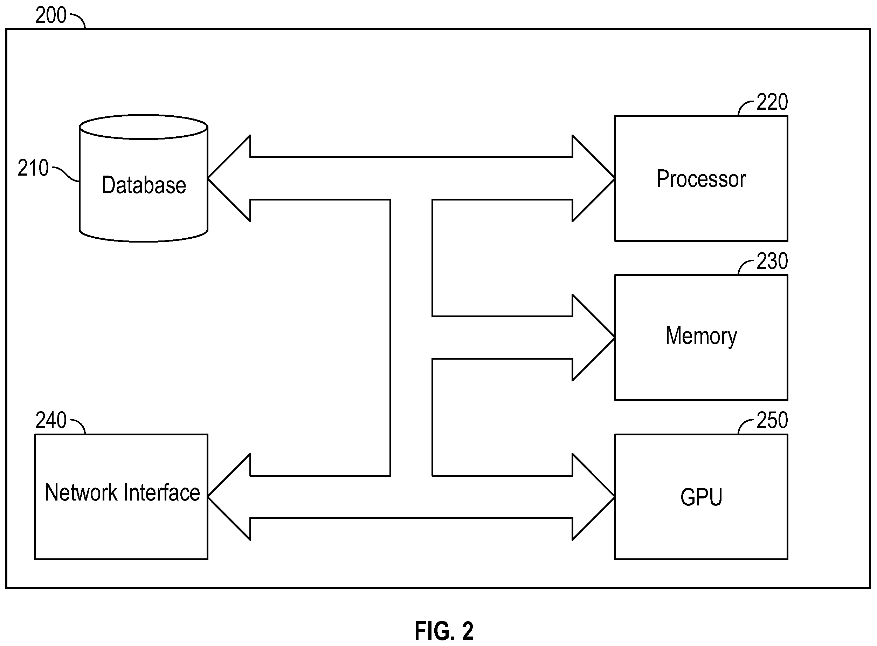

FIG. 1 illustrates an EndoFLIP system 100 according to this disclosure. The EndoFLIP system 100 generally includes a system 200, a display 104, a touch screen display 106, and a balloon catheter 108. The system 200 (FIG. 2) is configured to execute processor-readable instructions for data acquisition and analysis. Various balloon catheter configurations may be used depending on the application, size, and catheter diameter. The EndoFLIP system 100 may include a microphone 109 configured for voice command entry.

The EndoFLIP system 100 enables evaluation of the motor functions of an esophagus and/or other body lumen. The EndoFLIP system 100 provides useful information to support diagnosis of conditions like dysphagia, achalasia, and hiatal hernia. By precisely quantifying the contractions of the esophagus and its sphincters, this procedure helps provide a more complete esophageal pressure profile of the patient.

Esophageal measurements can be used to assess motility function of the esophagus and bolus transit dynamics in the esophagus. The balloon catheter 108 generally includes one or more sensors 112 (e.g., sixteen electrical sensors) disposed along the length of the balloon catheter 108 and configured to measure electrical impedance during a procedure. The one or more sensors 112 are and in communication with the system 200 and encapsulated in a balloon 110, which is configured to be inflated during a procedure. During a procedure, the balloon catheter 108 is inserted into the esophagus, typically reaching the lower esophageal sphincter (LES) and extending into the stomach of a patient, with the sensors 112 positioned at a plurality of points along the length of the esophagus. The LES is a muscle that separates the esophagus from the stomach and acts like a valve that normally stays tightly closed to prevent contents in the stomach from backing up into the esophagus.

During a procedure, the patient swallows a specific amount of water (or other bolus) with the balloon catheter 108 placed in the esophagus. The EndoFLIP system 100 inflates the balloon 110 to a specific volume (e.g., 30 mL) and records impedance measurements from the sensors 112 to create, for display, real-time images depicting estimated endoluminal diameter along the length of the body lumen (e.g., esophagus). In this way, the electrical impedance at the sensors 112 can be measured and used as an indication of the magnitude and sequence of the peristaltic contractions within the body lumen. In addition, because the positions of the sensors 112 along the catheter are prearranged, the velocity of the peristaltic motion can also be ascertained. The measurements may be repeated a number of times to obtain a set of endoluminal diameter and velocity values, a statistical analysis of which may be used for diagnostic purposes.

The impedance measurements provide for spatiotemporal plotting of bolus movement. Electrical impedance at a plurality of points in the esophagus can be used to detect and monitor the movement of a bolus through the esophagus. A bolus of water or food will cause the esophagus to have a different electrical impedance than that of a non-filled esophagus, such that a detected change in impedance in the esophagus is indicative of the presence of a bolus. With this in mind, the balloon catheter 108 positioned in the esophagus with a plurality of impedance sensors disposed along its length can be used to detect and monitor bolus transit, e.g., the movement of a bolus through the esophagus.

FIG. 1 is merely an example of an EndoFLIP system. Variations of the EndoFLIP system described herein are contemplated to be within the scope of the present disclosure.

FIG. 2 is a block diagram of an example of components in a system 200, which may be the EndoFLIP system 100 of FIG. 1. The system 200 includes a processor 220 that is connected to a processor-readable storage medium or a memory 230. The processor-readable storage medium or memory 230 may be a volatile type memory, *e.g.,* RAM, or a non-volatile type memory, *e.g.*, flash media, disk media, *etc.* In various aspects of the disclosure, the processor 220 may be any type of processor such as, without limitation, a digital signal processor, a microprocessor, an ASIC, a field-programmable gate array (FPGA), or a central processing unit (CPU), among other possible processors.

In aspects of the disclosure, the memory 230 can be random access memory, read-only memory, magnetic disk memory, solid-state memory, optical disc memory, and/or another type of memory. In some aspects of the disclosure, the memory 230 can be separate from the system 200 and can communicate with the processor 220 through communication buses of a circuit board. The memory 230 includes processor-readable instructions that are executable by the processor 220 to operate the system 200. The memory 230 may include volatile (e.g., RAM) and non-volatile storage configured to store data, including processor-readable instructions for operating the EndoFLIP system 100. In other aspects of the disclosure, the system 200 may include a network interface 240 to communicate with other computers or to a server. A database 210 and/or a storage device may be used for storing data.

FIG. 2 is merely an example. Persons skilled in the art will understand that the system 200 may include other components not illustrated in FIG. 2. In various embodiments, the system 200 may not include every component illustrated in FIG. 2. For example, the system 200 may not include a graphics processing unit 250. As another example, the system 200 may include an electronic storage but may not include a database 210. Such and other embodiments are within the scope of the present disclosure.

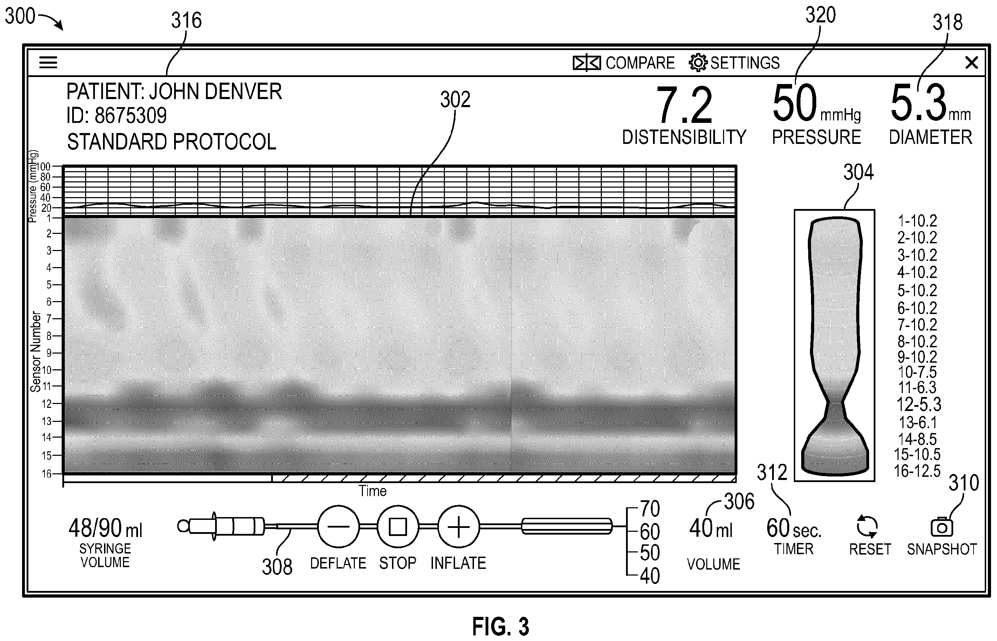

FIG. 3 shows an example of an interface 300 displaying a real-time 3D image generated by the EndoFLIP system of FIG. 1 based on the impedance measurements received from the sensors 112 disposed on the balloon catheter 108. The interface 300 generally includes a first image window 304 which displays a real-time 3D image depicting the endoluminal diameter along the balloon catheter 108 based on impedance measurements at the sensors 112, a second image window 302 which displays in a two-dimensional (2-D) representation of endoluminal diameter based on impedance measured at each of the sensors 112 over time. The 2D image displayed in the second image window 302 may continuously or periodically scroll horizontally (e.g., from right to left) as time elapses during the procedure. The interface 300 also includes a capture image control 310 configured for capturing a real-time image of the first and/or second image windows 302, 304, controls for inflating or deflating the balloon 308, an indication of the inflation setting 306, a timer 312, an indication of the measured pressure 320 of the body lumen (e.g., the esophagus), an indication of the diameter of the body lumen 318, and patient identification data 316. As shown in FIG. 3, the first image window 304 and the second image window 302 may be vertically aligned such that each of the endoluminal positions shown in one image window align vertically with the corresponding endoluminal positions shown in the other image window. For example, and with reference to FIG. 3, a sensor referenced as "12" in the second image window 302 vertically aligns with the same sensor referenced as "12" in the first image window 304 such that the endoluminal diameter at sensor "12" can be viewed by the clinician side-by-side in the 3D and 2D representations of the endoluminal diameter.

The description of FIG. 3 is merely an example. Variations of the graphical user interface are contemplated to be within the scope of the present disclosure.

Various aspects of an EndoFLIP system were described above. The following will describe image processing techniques for processing a 2D image, such as a 2D image in the second image window 302 of FIG. 3, to estimate a location of an esophago-gastric junction relative to the 2D image. The esophago-gastric junction (EGJ) is the junction between the esophagus and the stomach and is at the lower end of the esophagus. The EGJ has a lower boundary where the lowest point of the EGJ borders the highest point of the stomach.

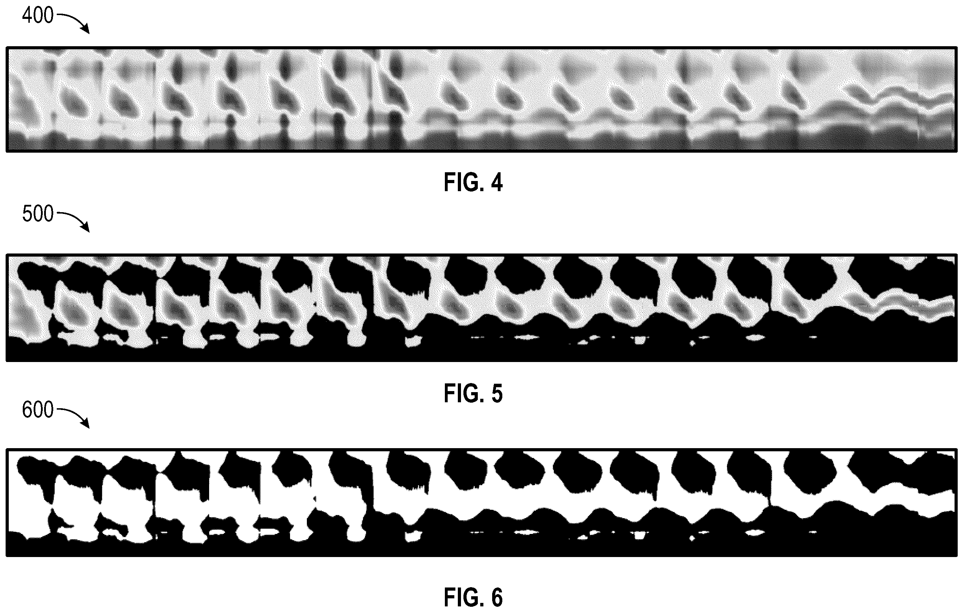

FIG. 4 is an example of a two-dimensional (2D) image 400 having a first dimension representative of endoluminal positions, a second dimension representative of points in time, and image pixels having pixel values indicative of endoluminal diameter, in accordance with aspects of the disclosure. The 2D image is generated by an EndoFLIP system and may be a 2D image in the second image window 302 of FIG. 3, for example. In the example of FIG. 4, points along the vertical dimension are representative of endoluminal positions, and points along the horizontal dimension are representative of points in time.

In the embodiment of FIG. 4, the vertical dimension includes interpolated pixels. As described in connection with FIG. 1 and FIG. 3, the EndoFLIP system includes impedance sensors, such as sixteen sensors, as shown in FIG. 3. Each sensor provides one estimate of endoluminal diameter. Therefore, an EndoFLIP system having sixteen sensors would provide sixteen original vertical pixels corresponding to the sixteen sensors. To provide a greater number of pixels in the vertical dimension of the 2D image 400, pixels between the original vertical pixels would be interpolated. Persons skilled in the art will understand how to implement such interpolation. Similarly, the measurements of the sixteen sensors are captured at a measurement rate, such as a rate of ten hertz or another rate. Accordingly, the number of pixels in the horizontal direction corresponds to the number of measurements taken over time at the measurement rate. Pixels in the horizontal dimension may be interpolated, as well, and persons skilled in the art will understand how to implement such interpolation.

The pixels of the 2D image 400 have pixel values that indicate endoluminal diameter. In various embodiments, the pixels of the 2D image 400 have values based on a color space, such as an RGB (red, green, blue) color space, an HSV (hue, saturation, value) color space, or another color space. In such embodiments, the color of a pixel (as indicated by the pixel's color space values) is indicative of endoluminal diameter. For example, a blue pixel color may indicate a large endoluminal diameter, a red pixel color may indicate a small endoluminal diameter, and other pixel colors may indicate endoluminal diameters between the blue and red colors. In various embodiments, different shades or brightness may be indicative of endoluminal diameters. Other embodiments of pixel values indicative of endoluminal diameters are contemplated to be within the scope of the present disclosure.

The 2D image 400 of FIG. 4 is an example of a color image in which different colors are indicative different endoluminal diameters. Although FIG. 4 shows the 2D image 400 in grayscale, the following description will refer to the 2D image 400 as a color image. In various embodiments, the 2D image 400 uses an RGB color space. In various embodiments, the 2D image 400 is converted from an RGB color space to an HSV color space. Additionally, the 2D image 400 is an example of an image having interpolated pixels.

The following description will refer FIGS. 5-11 as examples of image processing in accordance with aspects of the present disclosure.

Referring to FIG. 5, there is shown an example of the 2D image of FIG. 4 with a subset of pixels converted to black pixels, in accordance with aspects of the disclosure. The subset of pixels that are converted to black pixels are pixels whose pixel values indicate endoluminal diameters that are outside a predetermined range of endoluminal diameters. In various embodiments, the subset of pixels that are converted to black pixels may be pixels whose pixel values are outside a predetermined range of pixel values that correspond to the predetermined range of endoluminal diameters. In various embodiments, the image processing of FIG. 5 converts pixels which do not represent esophageal contraction into black pixels. The resulting 2D image 500 includes the black pixels and includes another subset of pixels whose pixel values represent endoluminal diameter within the predetermined range of endoluminal diameters.

FIG. 6 is an example of the 2D image of FIG. 5 with the other subset of pixels converted to white pixels, in accordance with aspects of the disclosure. As mentioned above, the other subset of pixels has pixel values that represent endoluminal diameter within the predetermined range of endoluminal diameters. In various embodiments, the image processing of FIG. 6 converts pixels which represent esophageal contraction into white pixels. The resulting 2D image 600 includes only the black pixels and the white pixels resulting from applying the image processing of FIG. 5 and FIG. 6. The image processing of FIG. 5 and FIG. 6 may be performed in sequence or performed simultaneously and may be performed in any order.

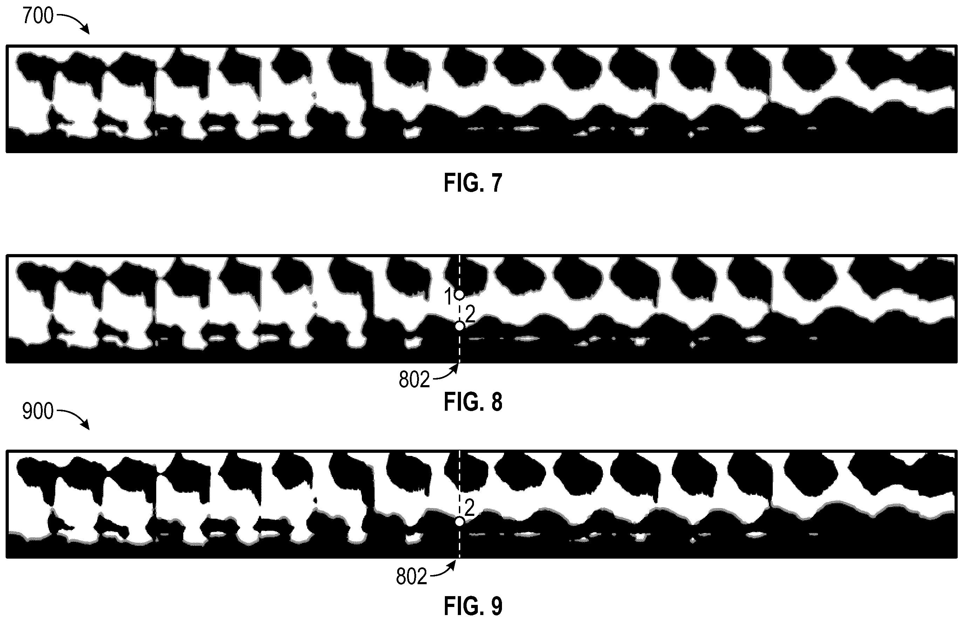

FIG. 7 is an example of the 2D image of FIG. 6 with illustrated contours of the black pixels, in accordance with aspects of the disclosure. A contour detection technique can be applied to the 2D image 600 of FIG. 6 to detect the contours of the black pixels. An example of a contour detection technique is described in S. Suzuki et al., "Topological structural analysis of digitized binary images by border following", Computer Vision, Graphics, and Image Processing, 30(1):32-46, 1985, which is hereby incorporated by reference herein in its entirety. Other contour or boundary detection techniques may be applied and are within the scope of the present disclosure. The detected contours are shown in FIG. 7 for illustration. In various embodiments, the result of the processing of FIG. 7 may be a collection of contour pixels which are specified by their pixel coordinates. In various embodiments, the contour pixels may be replaced by pixels of a particular color, such as green pixels, for example, which can result in the 2D image 700 of FIG. 7. Other manners of representing the contours and contour pixels are contemplated to be within the scope of the present disclosure.

FIG. 8 is an example of the 2D image of FIG. 7 in which contour points for a single point of time are determined, in accordance with aspects of the disclosure. In accordance with aspects of the present disclosure, the lowest contour point is identified for each time point along the horizontal dimension. In the example of FIG. 8, the time point 802 has two contour pixels - a higher contour pixel 1 and a lower contour pixel 2. Then, the processing of FIG. 9 operates to maintain the lowest contour pixel for each time point along the horizontal dimension. In the example of FIG. 9, the lower contour pixel 2 is maintained and all higher contour pixels are discarded or converted to white pixels.

In various embodiments, the processing of FIG. 8 and FIG. 9 may process the collection of contour pixels and the pixel coordinates described in connection with FIG. 7. In such embodiments, contour points having the same horizontal (time point) coordinate are aggregated, and the contour point having the lowest vertical (endoluminal position) coordinate is maintained, while the other contour points for the time point are discarded. The result of such processing is a collection of lowest contour pixels for each time point.

In various embodiments, the processing of FIG. 8 and FIG. 9 may perform image processing on the 2D image 700 of FIG. 7 to identify the lowest contour pixel for each time point. The lowest contour pixel may be identified by image processing, for example, that identifies the lowest pixel having a particular color (such as the lowest green pixel). The image processing may convert all higher contour pixels for each time point to white pixels and may result in the 2D image 900 of FIG. 9.

Other manners of processing the contours and contour pixels to identify the lowest contour pixel for each time point are contemplated to be within the scope of the present disclosure.

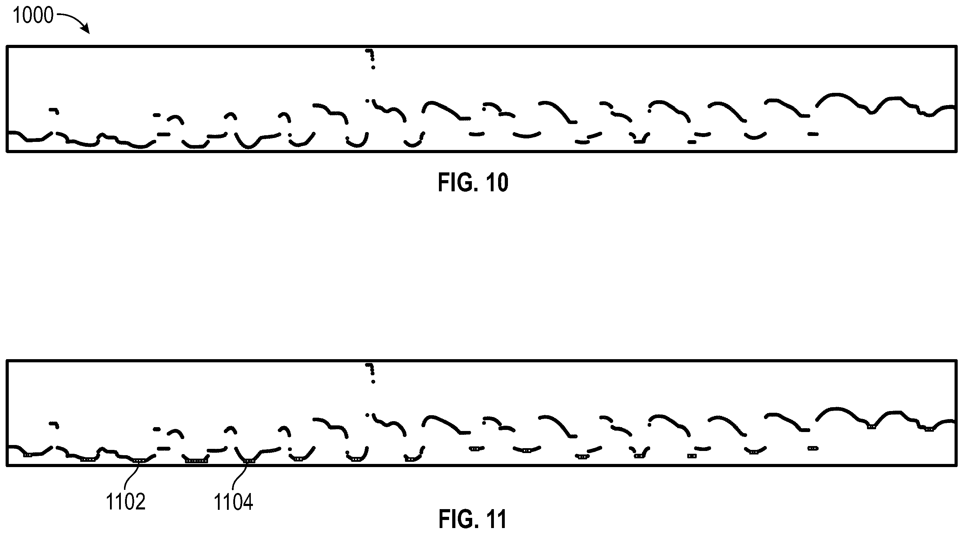

FIG. 10 is an example of the 2D image of FIG. 9 in which the lowest contour point for every point of time is shown, in accordance with aspects of the disclosure. In embodiments which maintain a collection of lowest contour points, the image of FIG. 10 is not needed. In embodiments which utilize image processing, the processing of FIG. 10 may convert all pixels in the 2D image 900 of FIG. 9 which are not contour pixels (e.g., do not have pixel color or pixel values indicative of a contour pixel) to white pixels, resulting in the 2D image 1000 of FIG. 10.

FIG. 11 is an example of the 2D image of FIG. 10 in which local minimum points are detected among the lowest contour points, in accordance with aspects of the disclosure. In accordance with aspects of the present disclosure, a local minimum detection technique can be applied to the lowest contour points of FIG. 10 to detect local minimum points of the contour points. Various local minimum detection techniques may be utilized. In various embodiments, a contour point may be selected as a local minimum if its K-direct neighbors have a larger or equal value. The value of the parameter K may be configurable and may be optimized based on, for example, the parameters of the EndoFLIP procedure and/or the physiology of a person, among other factors. In various embodiments, image processing techniques for identifying local minimums may be utilized. Such and other embodiments are contemplated to be within the scope of the present disclosure. Examples of local minimum points 1102, 1104 are shown in FIG. 11.

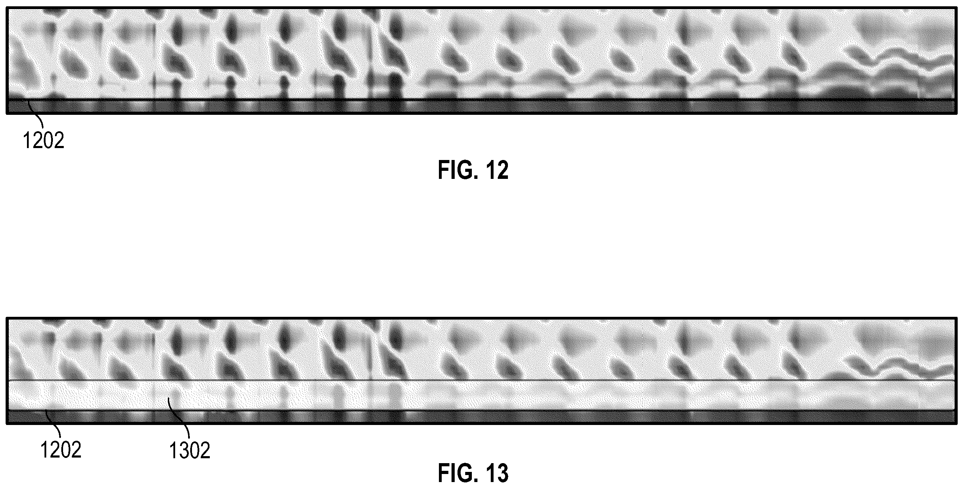

FIG. 12 is an example of the 2D image of FIG. 4 in which an estimated lower boundary location 1202 of the esophago-gastric junction is shown, in accordance with aspects of the disclosure. In accordance with aspects of the present disclosure, the lower boundary location of the esophago-gastric junction can be estimated based on the local minimum points resulting from the processing of FIG. 11. In various embodiments, the lower boundary location of the esophago-gastric junction may be estimated by gathering a predetermined percentage (such as 10%) or a predetermine number (e.g., 10) of the local minimum points having lowest vertical-dimension-coordinates, and determining the mean vertical-dimension-coordinate-value of the gathered local minimum points to be the estimated lower boundary location of the esophago-gastric junction. In various embodiments, the percentage may be a percentage different from 10%. In various embodiments, the predetermine number may be a number different from ten. In various embodiments, the gathered local minimum points may be processed in various other ways to provide the estimated lower boundary location of the esophago-gastric junction. Such other techniques may include, for example, discarding outliers, determining a mode of the gathered local minimum points, and/or weighting various local minimum points and determining a weighted average, among other techniques. Such and other techniques are contemplated to be within the scope of the present disclosure for estimating a lower boundary location of the esophago-gastric junction.

FIG. 13 is an example of the 2D image of FIG. 12 in which an estimated location of the esophago-gastric junction is illustrated, in accordance with aspects of the disclosure. As mentioned in connection with FIG. 4, the 2D image 400 of FIG. 4 may include interpolated pixels or may not include interpolated pixels. In both cases, the esophago-gastric junction may span multiple pixels of the 2D image 400 of FIG. 4. In various embodiments, where sixteen sensors are used in the EndoFLIP system of FIG. 1, for example, the esophago-gastric junction may span four sensors. A band 1302 or other indicator may be displayed relative to the 2D image 400 to indicate an estimated location of the esophago-gastric junction. The size of the band 1302 may be determined based on the number and arrangement of sensors in the EndoFLIP system of FIG. 1 and may be determined based on the amount of interpolation in the 2D image 400.

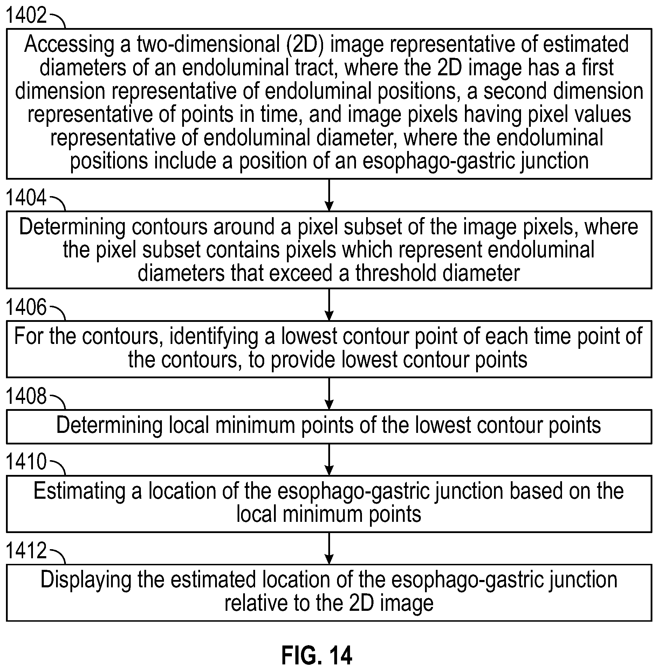

Various processing and 2D imaging resulting from such processing were described above in connection with FIGS. 4-13. The following describes an example of a processing operation with reference to FIG. 14.

FIG. 14 is a flow chart of an example of a processing operation. In various embodiments, the operation may be performed by the EndoFLIP system of FIG. 1. In various embodiments, the operation may be performed by a cloud computing system that communicates with the EndoFLIP system of FIG. 1.

At block 1402, the operation involves accessing a two-dimensional (2D) image representative of estimated diameters of an endoluminal tract, where the 2D image has a first dimension representative of endoluminal positions, a second dimension representative of points in time, and image pixels having pixel values representative of endoluminal diameter, where the endoluminal positions include a position of an esophago-gastric junction. The 2D image may be, for example, the image in the second image window 302 of FIG. 3 and may be an image such as the example shown in FIG. 4.

At block 1404, the operation involves determining contours around a pixel subset of the image pixels, where the pixel subset contains pixels which represent endoluminal diameters that exceed a threshold diameter. The processing at block 1404 may include the processing described in connection with FIGS. 5-7.

At block 1406, the operation involves, for the contours, identifying a lowest contour point of each time point of the contours, to provide lowest contour points. The processing at block 1406 may include the processing described in connection with FIGS. 8 and 9.

At block 1408, the operation involves determining local minimum points of the lowest contour points. The processing at block 1408 may include the processing described in connection with FIG. 10.

At block 1410, the operation involves estimating a location of the esophago-gastric junction based on the local minimum points. The processing at block 1410 may include the processing described in connection with FIGS. 12 and 13.

At block 1412, the operation involves displaying the estimated location of the esophago-gastric junction relative to the 2D image. An example of a display showing the estimated location of the esophago-gastric junction relative to the 2D image is shown in FIG. 13.

The operation of FIG. 14 is merely an example. Variations are contemplated to be within the scope of the present disclosure. For example, in various embodiments, the operation may include blocks not shown in FIG. 14. Such and other variations are within the scope of the present disclosure.

Various aspects of an EndoFLIP system were described above. The following will describe a graphical user interface (GUI) for use with EndoFLIP data, such as with the 2D image in the second image window 302 of FIG. 3, to mark the location of an anatomical feature (e.g., esophago-gastric junction) and/or mark timing and duration of certain events (e.g., filling volume event, dry catheter artifact event) and/or of certain features or characteristics (e.g., pressure peaks). Persons skilled in the art will understand the anatomical features, events, features, and characteristics.

As mentioned above, the esophago-gastric junction (EGJ) is the junction between the esophagus and the stomach and is at the lower end of the esophagus. The EGJ has a lower boundary where the lowest point of the EGJ borders the highest point of the stomach. In various embodiments, the EGJ spans multiple sensors of the EndoFLIP system. A filling volume event refers to the filling of the catheter of FIG. 1 with liquid. The catheter may be filled with, for example, 60 mL or 70 mL of liquid, among other volumes. A dry catheter artifact event refers to a data artifact that can occur when a contraction on the catheter interrupts conductivity between the catheter's electrodes, which results in artifactually large diameter measurements at the EGJ and/or esophageal body. Data during a filling volume event and data affected by dry catheter artifact should be excluded. Pressure peaks refer to local maximum points in the pressure data obtained by the EndoFLIP system.

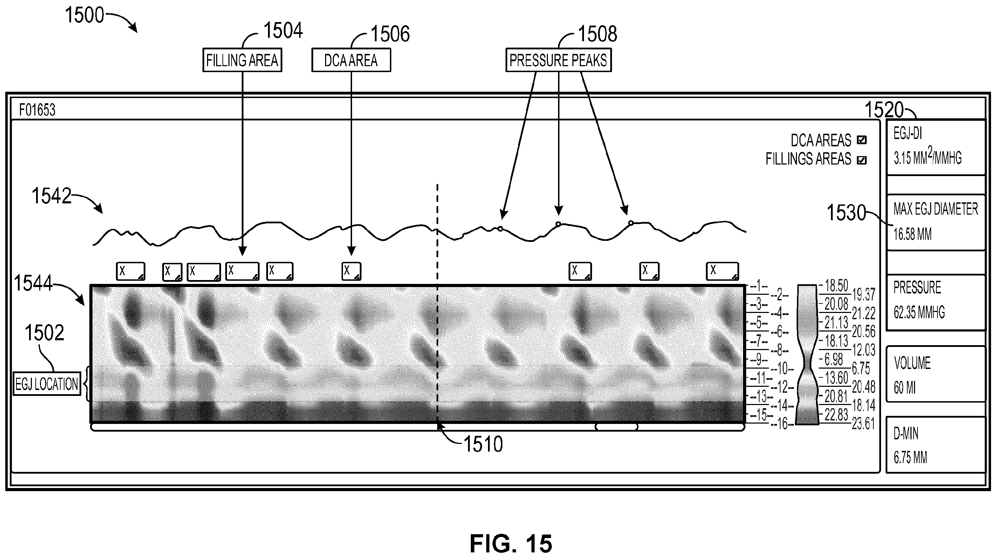

FIG. 15 shows an example of a graphical user interface (GUI) in accordance with aspects of the present disclosure. The GUI 1500 of FIG. 15 may be provided in a web browser or in an app or application. The GUI 1500 includes EndoFLIP data, including pressure data 1542 and a 2D image 1544, which may be a 2D image as described in connection with FIG. 3. The GUI 1500 displays various values, including a distensibility index 1520 and a maximum EGJ diameter 1530, among other values, such as pressure, volume, and minimum lumen diameter at a particular time point 1510 in the EndoFLIP data.

Distensibility index (DI) 1520 may be computed as follows:

$$DI = \frac{\text{median (CSA values at 3 pressure peaks of 60mL)}}{\text{median (Pressure values at 3 pressure peaks of 60mL)}} \qquad (1)$$

In the DI computation, the three (3) pressure peaks of 60mL refer to the three highest pressure peaks based on filling the catheter of the EndoFLIP system with 60 mL of liquid. The pressure values at the three pressure peaks are self-explanatory. The CSA refers to the cross-sectional area of the lumen at the minimum diameter along the lumen, between the bottom and the top of the marked EGJ, during the three pressure peaks. These values are used to compute the distensibility index 1520 as shown in computation (1) above.

The maximum EGJ diameter 1530 may be computed as follows:

$$\begin{aligned}
\text{Maximum EGJ diameter} = \\
\max( \\
\text{median(minimum diameter values at 3 pressure peaks of 60ml),} \qquad (2) \\
\text{median(minimum diameter values at 3 pressure peaks of 70ml)} \\
)
\end{aligned}$$

In the maximum EGJ diameter computation, the three (3) pressure peaks of 60mL refer to the three highest pressure peaks based on filling the catheter of the EndoFLIP system with 60 mL of liquid. The three (3) pressure peaks of 70mL refer to the three highest pressure peaks based on filling the catheter of the EndoFLIP system with 70 mL of liquid. The minimum diameter value at each pressure peak refers to the minimum diameter along the lumen, between the bottom and the top of the marked EGJ, during the pressure peak. These values are used to compute the maximum EGJ diameter 1530 as shown in computation (2) above.

The GUI 1500 includes various user interface elements 1502-1508. A user interface element 1502 marks the location of the EGJ and can be adjusted by a user to adjust the marked location of the EGJ. The user interface element 1502 may have a rectangular shape, such as a white transparent rectangle that is located within the 2D image 1544, as shown in FIG. 15. Other configurations for the user interface element 1502 are contemplated to be within the scope of the present disclosure.

One or more user interface elements 1504 mark the starting time and duration of a filling volume event and can be added, moved, adjusted, and/or removed by a user. The user interface element 1504 may have a rectangular shape, such as a pink rectangle that is located above the 2D image 1544. The start of the rectangle marks the start of the filling volume event, and the end of the rectangle marks the end of the filling volume event. The rectangle can be moved and adjusted to mark an event start time, an event end time, and an event duration. Other configurations for the user interface element 1504 are contemplated to be within the scope of the present disclosure.

One or more user interface elements 1506 mark the starting time and duration of a dry catheter artifact (DCA) feature and can be added, moved, adjusted, and/or removed by a user. The user interface element 1506 may have a rectangular shape, such as a gray rectangle that is located above the 2D image 1544. The start of the rectangle marks the start of the DCA event, and the end of the rectangle marks the end of the DCA event. The rectangle can be moved and adjusted to mark an event start time, an event end time, and an event duration. Other configurations for the user interface element 1506 are contemplated to be within the scope of the present disclosure.

One or more user interface elements 1506 mark the starting time and duration of a dry catheter artifact (DCA) feature and can be added, moved, adjusted, and/or removed by a user. The user interface element 1506 may have a rectangular shape, such as a gray rectangle that is located above the 2D image 1544. The start of the rectangle marks the start of the DCA event, and the end of the rectangle marks the end of the DCA event. The rectangle can be moved and adjusted to mark an event start time, an event end time, and an event duration. Other configurations for the user interface element 1506 are contemplated to be within the scope of the present disclosure.

One or more user interface elements 1508 mark the locations of pressure peaks and can be added, moved, adjusted, and/or removed by a user. The user interface element 1508 may be in the form of a dot, such as a red dot that is located directly on the pressure data 1542. The dot can be moved to mark a pressure peak. Other configurations for the user interface element 1508 are contemplated to be within the scope of the present disclosure.

In various embodiments, the user interface elements 1502-1508 may be added or removed by menu options or selectable commands or other commands (not shown). In various embodiments, the user interface elements 1502-1508 may be adjusted or moved by dragging using a mouse or using a user's finger (in the case of a touchscreen interface) or may be adjusted by entering parameters using an input box (not shown).

In various embodiments, the GUI 1500 may be used to display EndoFLIP data for a filling volume of 60 mL and to display EndoFLIP data for a filling volume of 70 mL. A user may switch between displaying the EndoFLIP data for 60 mL and the EndoFLIP data for 70 mL using, e.g., a menu option, separate display windows, or using another manner (not shown). Each display may include user interface elements 1502-1508 corresponding to the displayed data.

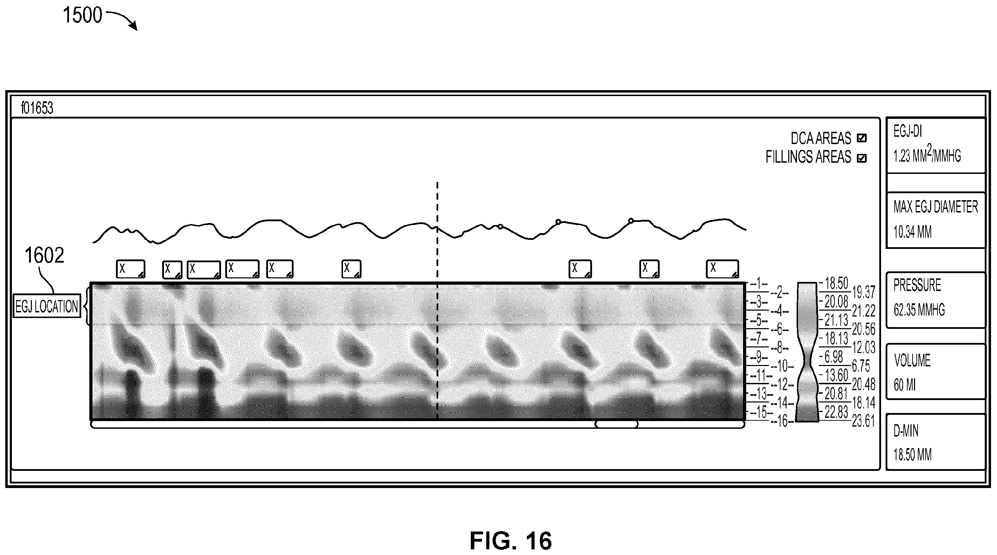

The user interface elements 1502-1508 affect the DI computation (1) and the maximum EGJ diameter computation (2), which will be described in connection with FIGS. 16-18.

The GUI of FIG. 15 is merely an example, and variations are contemplated to be within the scope of the present disclosure.

FIG. 16 is a diagram of the GUI 1500 of FIG. 15 in which the user interface element indicating the marked location of an esophago-gastric junction is moved by a user to a new location 1602. In response to the new marked location of the EGJ, the DI computation (1) and the maximum EGJ diameter computation (2) can be performed to update the DI and maximum EGJ diameter values displayed in the GUI 1500. For example, the new marked location of the EGJ may affect the value of the minimum diameter of the lumen between the bottom and the top of the marked location of the EGJ. As the EGJ is moved upward or downward, different ranges of the lumen are usable for determining such minimum diameter; i.e., the lumen range between the bottom and the top of the marked location of the EGJ is usable data for determining such minimum diameter.

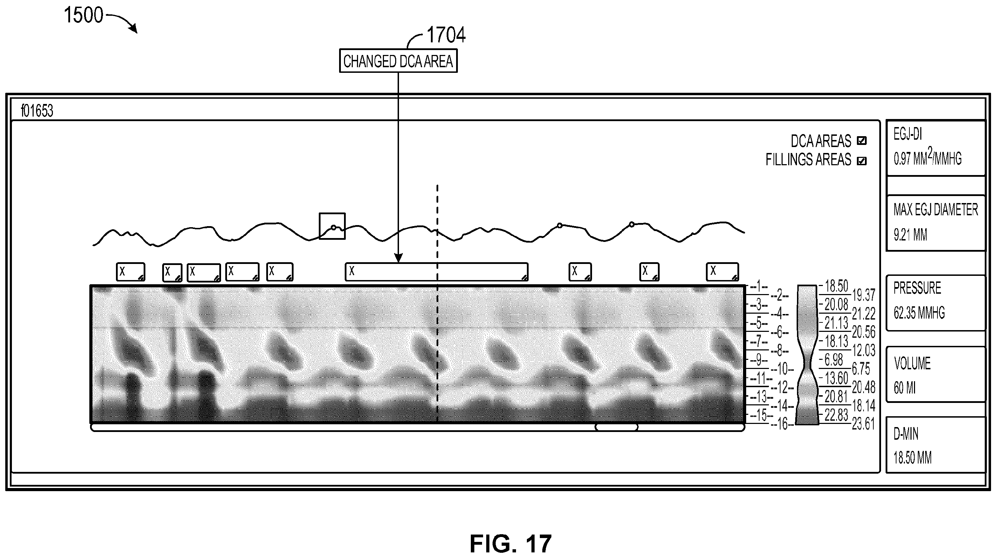

FIG. 17 is a diagram of the GUI 1500 of FIG. 15 in which a user interface element indicating a marked duration of dry catheter artifact (DCA) is moved and/or adjusted by a user to a different start time and/or different duration 1704. In various embodiments, the user interface element can be deleted by engaging the "x" shown in the user interface element. In various embodiments, a user interface element can be added to mark a new DCA event. In response to the new, deleted, or different marked time and/or duration of the DCA, the DI computation (1) and the maximum EGJ diameter computation (2) can be performed to update the DI and maximum EGJ diameter values displayed in the GUI 1500. For example, a new or different marked time and/or duration 1704 of the DCA indicates data to be excluded from being usable for the computations. A deleted user interface element makes more EndoFLIP data usable for the computations. As the user interface element is added, deleted, moved, and/or adjusted, different ranges of the EndoFLIP data are usable for such computations. The same operation applies to the marked time and/or duration of the filling volume events, as well.

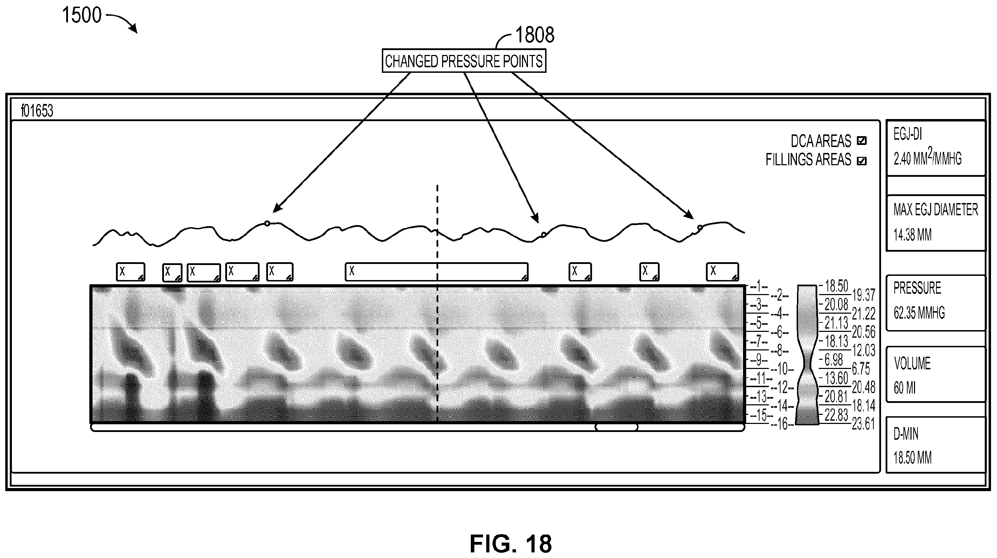

FIG. 18 is a diagram of the GUI 1500 of FIG. 15 in which a user interface element indicating a marked pressure peak is adjusted by a user to a new pressure point 1808. In response to the new marked pressure point, the DI computation (1) and the maximum EGJ diameter computation (2) can be performed to update the DI and maximum EGJ diameter values displayed in the GUI 1500. For example, the new marked pressure point 1808 indicates a pressure point that is usable for the computations. As pressure points are moved, added, or removed, different EndoFLIP data are usable for such computations. In various embodiments, a user can indicate more than three pressure points, and the three highest among the marked pressure points may be used for the computations. In various embodiments, initial pressure peaks may be identified by a processing technique such a local maximum identification technique, without human intervention. A user may then move or remove such pressure points determined by the processing technique.

The description of FIGS. 15-18 are merely examples. In various embodiments, different numbers of user interface elements may be used in a GUI. Such and other variations are contemplated to be within the scope of the present disclosure.

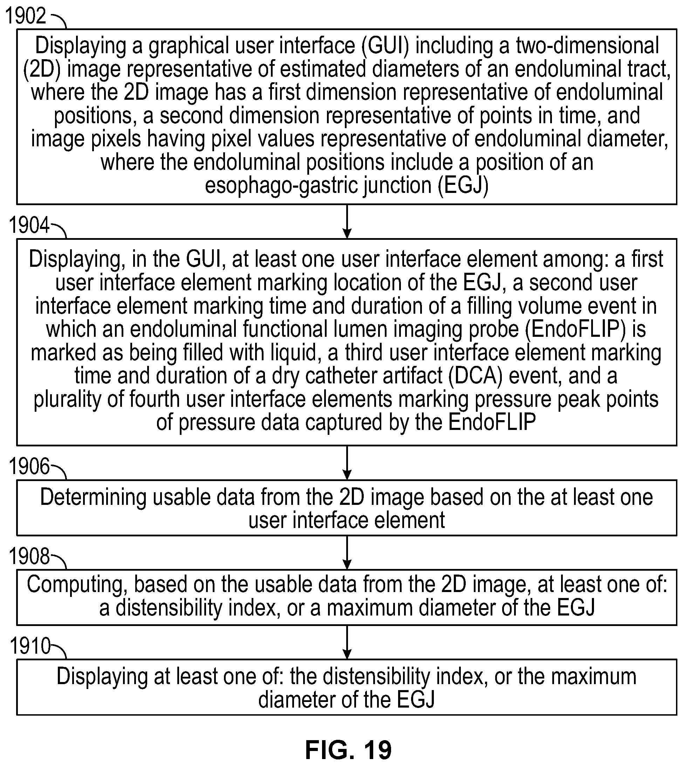

Various graphical user interface elements and associated operations were described above in connection with FIGS. 15-18. The following describes an example of a processing operation with reference to FIG. 19.

FIG. 19 is a flow chart of an example of a processing operation. In various embodiments, the operation may be performed by the EndoFLIP system of FIG. 1. In various embodiments, the operation may be performed by a cloud computing system that communicates with the EndoFLIP system of FIG. 1.

At block 1902, the operation involves displaying a graphical user interface (GUI) including a two-dimensional (2D) image representative of estimated diameters of an endoluminal tract, where the 2D image has a first dimension representative of endoluminal positions, a second dimension representative of points in time, and image pixels having pixel values representative of endoluminal diameter, where the endoluminal positions include a position of an esophago-gastric junction (EGJ).

At block 1904, the operation involves displaying, in the GUI, at least one user interface element among: a first user interface element marking location of the EGJ, a second user interface element marking time and duration of a filling volume event in which an endoluminal functional lumen imaging probe (EndoFLIP) is marked as being filled with liquid, a third user interface element marking time and duration of a dry catheter artifact (DCA) event, and a plurality of fourth user interface elements marking pressure peak points of pressure data captured by the EndoFLIP.

At block 1906, the operation involves determining usable data from the 2D image based on the at least one user interface element. For example, with respect to the first user interface element marking location of the EGJ, the usable data includes data in the 2D image between the bottom and the top of the marked location of the EGJ and excludes data in the 2D image above and data in the 2D image below the marked location of the EGJ. With respect to the second user interface element marking time and duration of a filling volume event and the third user interface element marking time and duration of a dry catheter artifact (DCA) event, the usable data of the 2D image excludes data in the 2D image encompassed by the time and duration marked by the second user interface element or by the time and duration marked by the third user interface element. With respect to the plurality of fourth user interface elements marking pressure peak points of pressure data captured by the EndoFLIP, the usable data of the 2D image includes data of the 2D image corresponding to the pressure points marked by the plurality of fourth user interface elements and excludes data of the 2D image that correspond to pressure points different from the pressure points marked by the plurality of fourth user interface elements.

At block 1908, the operation involves computing, based on the usable data from the 2D image, at least one of: a distensibility index, or a maximum diameter of the EGJ.

At block 1910, the operation involves displaying at least one of: the distensibility index, or the maximum diameter of the EGJ.

The operation of FIG. 19 is merely an example. Variations are contemplated to be within the scope of the present disclosure. For example, in various embodiments, the operation may include blocks not shown in FIG. 19. Such and other variations are within the scope of the present disclosure.

The following describes further aspects of the present disclosure.

Example 1.1. A system comprising:

at least one processor; and
at least one memory storing instructions which, when executed by the at least one processor, cause the system to at least perform:

displaying a graphical user interface (GUI) comprising a two-dimensional (2D) image representative of estimated diameters of an endoluminal tract, the 2D image having a first dimension representative of endoluminal positions, a second dimension representative of points in time, and image pixels having pixel values representative of endoluminal diameter, wherein the endoluminal positions comprise a position of an esophago-gastric junction (EGJ);
displaying, in the GUI, at least one user interface element among:

a first user interface element marking location of the EGJ,
a second user interface element marking time and duration of a filling volume event in which an endoluminal functional lumen imaging probe (EndoFLIP) is marked as being filled with liquid,
a third user interface element marking time and duration of a dry catheter artifact (DCA) event, and
a plurality of fourth user interface elements marking pressure peak points of pressure data captured by the EndoFLIP;

determining usable data from the 2D image based on the at least one user interface element;
computing, based on the usable data from the 2D image, at least one of: a distensibility index, or a maximum diameter of the EGJ; and
displaying at least one of: the distensibility index, or the maximum diameter of the EGJ.

Example 1.2. The system of Example 1.1, wherein the first user interface element marking the location of the EGJ is movable by a user to mark a new location of the EGJ, and
wherein the usable data of the 2D image excludes data in the 2D image above and data in the 2D image below the marked location of the EGJ.

Example 1.3. The system of Example 1.1 or Example 1.2, wherein the second user interface element marking the time and duration of a filling volume event is movable by a user to mark a different start time of the filling volume event, is adjustable by the user to mark a different duration of the filling volume event, and is deletable by the user, and
wherein the usable data of the 2D image excludes data in the 2D image encompassed by the time and duration marked by the second user interface element.

Example 1.4. The system of any one of Example 1.1- Example 1.3, wherein the third user interface element marking the time and duration of a DCA movable by a user to mark a different start time of the DCA event, is adjustable by the user to mark a different duration of the DCA event, and is deletable by the user, and
wherein the usable data of the 2D image excludes data in the 2D image encompassed by the time and duration marked by the third user interface element.

Example 1.5. The system of any one of Example 1.1- Example 1.4, wherein the plurality of fourth user interface elements marking the pressure peak points are movable by a user to mark different pressure peak points and are deletable by a user, and
wherein the usable data of the 2D image excludes data of the 2D image that correspond to pressure points different from the pressure points marked by the plurality of fourth user interface elements.

Example 1.6. A processor-implemented method comprising:

displaying a graphical user interface (GUI) comprising a two-dimensional (2D) image representative of estimated diameters of an endoluminal tract, the 2D image having a first dimension representative of endoluminal positions, a second dimension representative of points in time, and image pixels having pixel values representative of endoluminal diameter, wherein the endoluminal positions comprise a position of an esophago-gastric junction (EGJ);

displaying, in the GUI, at least one user interface element among:

a first user interface element marking location of the EGJ,
a second user interface element marking time and duration of a filling volume event in which an endoluminal functional lumen imaging probe (EndoFLIP) is marked as being filled with liquid,
a third user interface element marking time and duration of a dry catheter artifact (DCA) event, and
a plurality of fourth user interface elements marking pressure peak points of pressure data captured by the EndoFLIP;

determining usable data from the 2D image based on the at least one user interface element;
computing, based on the usable data from the 2D image, at least one of: a distensibility index, or a maximum diameter of the EGJ; and
displaying at least one of: the distensibility index, or the maximum diameter of the EGJ.

Example 1.7. The processor-implemented method of claim Example 1.6, wherein the first user interface element marking the location of the EGJ is movable by a user to mark a new location of the EGJ, and
wherein the usable data of the 2D image excludes data in the 2D image above and data in the 2D image below the marked location of the EGJ.

Example 1.8. The processor-implemented method of Example 1.6 or Example 1.7, wherein the second user interface element marking the time and duration of a filling volume event is movable by a user to mark a different start time of the filling volume event, is adjustable by the user to mark a different duration of the filling volume event, and is deletable by the user, and
wherein the usable data of the 2D image excludes data in the 2D image encompassed by the time and duration marked by the second user interface element.

Example 1.9. The processor-implemented method of any one of Example 1.6-Example 1.8, wherein the third user interface element marking the time and duration of a DCA movable by a user to mark a different start time of the DCA event, is adjustable by the user to mark a different duration of the DCA event, and is deletable by the user, and
wherein the usable data of the 2D image excludes data in the 2D image encompassed by the time and duration marked by the third user interface element.

Example 1.10. The processor-implemented method of any one of Example 1.6-Example 1.9, wherein the plurality of fourth user interface elements marking the pressure peak points are movable by a user to mark different pressure peak points and are deletable by a user, and
wherein the usable data of the 2D image excludes data of the 2D image that correspond to pressure points different from the pressure points marked by the plurality of fourth user interface elements.

Example 1.11. A non-transitory processor-readable medium storing instructions which, when executed by at least one processor of a system, cause the system to at least perform:

displaying a graphical user interface (GUI) comprising a two-dimensional (2D) image representative of estimated diameters of an endoluminal tract, the 2D image having a first dimension representative of endoluminal positions, a second dimension representative of points in time, and image pixels having pixel values representative of endoluminal diameter, wherein the endoluminal positions comprise a position of an esophago-gastric junction (EGJ);

displaying, in the GUI, at least one user interface element among:

a first user interface element marking location of the EGJ,
a second user interface element marking time and duration of a filling volume event in which an endoluminal functional lumen imaging probe (EndoFLIP) is marked as being filled with liquid,
a third user interface element marking time and duration of a dry catheter artifact (DCA) event, and
a plurality of fourth user interface elements marking pressure peak points of pressure data captured by the EndoFLIP;

determining usable data from the 2D image based on the at least one user interface element;
computing, based on the usable data from the 2D image, at least one of: a distensibility index, or a maximum diameter of the EGJ; and
displaying at least one of: the distensibility index, or the maximum diameter of the EGJ.

Example 1.12. The non-transitory processor-readable medium of Example 1.11, wherein the first user interface element marking the location of the EGJ is movable by a user to mark a new location of the EGJ, and
wherein the usable data of the 2D image excludes data in the 2D image above and data in the 2D image below the marked location of the EGJ.

Example 1.13. The non-transitory processor-readable medium of Example 1.11 or Example 1.12, wherein the second user interface element marking the time and duration of a filling volume event is movable by a user to mark a different start time of the filling volume event, is adjustable by the user to mark a different duration of the filling volume event, and is deletable by the user, and
wherein the usable data of the 2D image excludes data in the 2D image encompassed by the time and duration marked by the second user interface element.

Example 1.14. The non-transitory processor-readable medium of any one of Example 1.11- Example 1.13, wherein the third user interface element marking the time and duration of a DCA movable by a user to mark a different start time of the DCA event, is adjustable by the user to mark a different duration of the DCA event, and is deletable by the user, and
wherein the usable data of the 2D image excludes data in the 2D image encompassed by the time and duration marked by the third user interface element.

Example 1.15. The non-transitory processor-readable medium of any one of Example 1.11- Example 1.14, wherein the plurality of fourth user interface elements marking the pressure peak points are movable by a user to mark different pressure peak points and are deletable by a user, and
wherein the usable data of the 2D image excludes data of the 2D image that correspond to pressure points different from the pressure points marked by the plurality of fourth user interface elements.

Example 2.1. A system comprising:

at least one processor; and
at least one memory storing instructions which, when executed by the at least one processor, causes the system to at least perform:

accessing a two-dimensional (2D) image representative of estimated diameters of an endoluminal tract, the 2D image having a first dimension representative of endoluminal positions, a second dimension representative of points in time, and image pixels having pixel values representative of endoluminal diameter, wherein the endoluminal positions comprise a position of an esophago-gastric junction;
determining contours around a pixel subset of the image pixels, the pixel subset containing pixels which represent endoluminal diameters that exceed a threshold diameter;
for the contours, identifying a lowest contour point of each time point of the contours, to provide lowest contour points;
determining local minimum points of the lowest contour points;
estimating a location of the esophago-gastric junction based on the local minimum points; and
displaying the estimated location of the esophago-gastric junction relative to the 2D image.

Example 2.2. The system of Example 2.1, wherein in the determining the contours around the pixel subset, the instructions, when executed by the at least one processor, cause the system to at least perform:

replacing pixel values of the 2D image which are outside a predetermined range, with new pixel values representative of a black pixel, wherein the pixel subset contains the black pixels; and
applying a contour detection technique to determine the contours around the black pixels.

Example 2.3. The system of Example 2.2, wherein the instructions, when executed by the at least one processor, further cause the system to at least perform:

replacing pixel values of the 2D image which are within the predetermined range, with new pixel values representative of a white pixel,
wherein the contour detection technique is applied to the 2D image containing only the black pixels and the white pixels.

Example 2.4. The system of Example 2.3, wherein the 2D image has an RGB color space,
wherein the instructions, when executed by the at least one processor, further cause the system to at least perform:
prior to the replacing the pixel values of the 2D image which are outside the predetermined range and which are within the predetermined range, converting the 2D image to an HSV color space.

Example 2.5. The system of Example 2.1, wherein in the estimating the location of the esophago-gastric junction based on the local minimum points, the instructions, when executed by the at least one processor, cause the system to at least perform:

gathering a predetermined percentage or predetermined number of the local minimum points to provide gathered local minimum points, the gathered local minimum points comprising lowest local minimum points among the local minimum points; and

determining a mean of the gathered local minimum points as a lower boundary location of the esophago-gastric junction.

Example 2.6. A processor-implemented method comprising:

accessing a two-dimensional (2D) image representative of estimated diameters of an endoluminal tract, the 2D image having a first dimension representative of endoluminal positions, a second dimension representative of points in time, and image pixels having pixel values representative of endoluminal diameter, wherein the endoluminal positions comprise a position of an esophago-gastric junction;

determining contours around a pixel subset of the image pixels, the pixel subset containing pixels which represent endoluminal diameters that exceed a threshold diameter;

for the contours, identifying a lowest contour point of each time point of the contours, to provide lowest contour points;

determining local minimum points of the lowest contour points;

estimating a location of the esophago-gastric junction based on the local minimum points; and

displaying the estimated location of the esophago-gastric junction relative to the 2D image.

Example 2.7. The processor-implemented method of Example 2.6, wherein the determining the contours around the pixel subset comprises:

replacing pixel values of the 2D image which are outside a predetermined range, with new pixel values representative of a black pixel, wherein the pixel subset contains the black pixels; and

applying a contour detection technique to determine the contours around the black pixels.

Example 2.8. The processor-implemented method of Example 2.7, further comprising:

replacing pixel values of the 2D image which are within the predetermined range, with new pixel values representative of a white pixel,

wherein the contour detection technique is applied to the 2D image containing only the black pixels and the white pixels.

Example 2.9. The processor-implemented method of Example 2.8, wherein the 2D image has an RGB color space,

the processor-implemented method further comprising:

prior to the replacing the pixel values of the 2D image which are outside the predetermined range and which are within the predetermined range, converting the 2D image to an HSV color space.

Example 2.10. The processor-implemented method of Example 2.6, wherein the estimating the location of the esophago-gastric junction based on the local minimum points comprises:

gathering a predetermined percentage or predetermined number of the local minimum points to provide gathered local minimum points, the gathered local minimum points comprising lowest local minimum points among the local minimum points; and

determining a mean of the gathered local minimum points as a lower boundary location of the esophago-gastric junction.

Example 2.11. A non-transitory processor-readable medium storing instructions which, when executed by at least one processor of a system, cause the system to at least perform:

accessing a two-dimensional (2D) image representative of estimated diameters of an endoluminal tract, the 2D image having a first dimension representative of endoluminal positions, a second dimension representative of points in time, and image pixels having pixel values representative of endoluminal diameter, wherein the endoluminal positions comprise a position of an esophago-gastric junction;

# EP 4 643 771 A1

determining contours around a pixel subset of the image pixels, the pixel subset containing pixels which represent endoluminal diameters that exceed a threshold diameter;

for the contours, identifying a lowest contour point of each time point of the contours, to provide lowest contour points;

determining local minimum points of the lowest contour points;

estimating a location of the esophago-gastric junction based on the local minimum points; and

displaying the estimated location of the esophago-gastric junction relative to the 2D image.

Example 2.12. The non-transitory processor-readable medium of Example 2.11, wherein in the determining the contours around the pixel subset, the instructions, when executed by the at least one processor, cause the system to at least perform:

replacing pixel values of the 2D image which are outside a predetermined range, with new pixel values representative of a black pixel, wherein the pixel subset contains the black pixels; and

applying a contour detection technique to determine the contours around the black pixels.

Example 2.13. The non-transitory processor-readable medium of Example 2.12, wherein the instructions, when executed by the at least one processor, further cause the system to at least perform:

replacing pixel values of the 2D image which are within the predetermined range, with new pixel values representative of a white pixel,

wherein the contour detection technique is applied to the 2D image containing only the black pixels and the white pixels.

Example 2.14. The non-transitory processor-readable medium of Example 2.13, wherein the 2D image has an RGB color space,

wherein the instructions, when executed by the at least one processor, further cause the system to at least perform: prior to the replacing the pixel values of the 2D image which are outside the predetermined range and which are within the predetermined range, converting the 2D image to an HSV color space.

Example 2.15. The non-transitory processor-readable medium of Example 2.11, wherein in the estimating the location of the esophago-gastric junction based on the local minimum points, the instructions, when executed by the at least one processor, cause the system to at least perform:

gathering a predetermined percentage or predetermined number of the local minimum points to provide gathered local minimum points, the gathered local minimum points comprising lowest local minimum points among the local minimum points; and

determining a mean of the gathered local minimum points as a lower boundary location of the esophago-gastric junction.

The embodiments disclosed herein are examples of the disclosure and may be embodied in various forms. For instance, although certain embodiments herein are described as separate embodiments, each of the embodiments herein may be combined with one or more of the other embodiments herein. Specific structural and functional details disclosed herein are not to be interpreted as limiting, but as a basis for the claims and as a representative basis for teaching one skilled in the art to variously employ the present disclosure in virtually any appropriately detailed structure. Like reference numerals may refer to similar or identical elements throughout the description of the figures.

The phrases "in an embodiment," "in embodiments," "in various embodiments," "in some embodiments," or "in other embodiments" may each refer to one or more of the same or different embodiments in accordance with the present disclosure. A phrase in the form "A or B" means "(A), (B), or (A and B)." A phrase in the form "at least one of A, B, or C" means "(A); (B); (C); (A and B); (A and C); (B and C); or (A, B, and C)."

The systems, devices, and/or servers described herein may utilize one or more processors to receive various information and transform the received information to generate an output. The processors may include any type of computing device, computational circuit, or any type of controller or processing circuit capable of executing a series of instructions that are stored in a memory. The processor may include multiple processors and/or multicore central processing units (CPUs) and may include any type of device, such as a microprocessor, graphics processing unit (GPU), digital signal processor, microcontroller, programmable logic device (PLD), field programmable gate array (FPGA), or the like. The processor may also include a memory to store data and/or instructions that, when executed by the one or more processors, causes the one or more processors to perform one or more methods and/or algorithms.

Any of the herein described methods, programs, algorithms or codes may be converted to, or expressed in, a programming language or computer program. The terms "programming language" and "computer program," as used herein, each include any language used to specify instructions to a computer, and include (but is not limited to) the following languages and their derivatives: Assembler, Basic, Batch files, BCPL, C, C+, C++, Delphi, Fortran, Java, JavaScript, machine code, operating system command languages, Pascal, Perl, PL1, Python, scripting languages, Visual Basic, metalanguages which themselves specify programs, and all first, second, third, fourth, fifth, or further generation computer languages. Also included are database and other data schemas, and any other meta-languages. No distinction is made between languages which are interpreted, compiled, or use both compiled and interpreted approaches. No distinction is made between compiled and source versions of a program. Thus, reference to a program, where the programming language could exist in more than one state (such as source, compiled, object, or linked) is a reference to any and all such states. Reference to a program may encompass the actual instructions and/or the intent of those instructions.

It should be understood that the foregoing description is only illustrative of the present disclosure. Various alternatives and modifications can be devised by those skilled in the art without departing from the disclosure. Accordingly, the present disclosure is intended to embrace all such alternatives, modifications and variances. The embodiments described with reference to the attached drawing figures are presented only to demonstrate certain examples of the disclosure. Other elements, steps, methods, and techniques that are insubstantially different from those described above and/or in the appended claims are also intended to be within the scope of the disclosure.

## Claims

1. A system comprising:

   at least one processor; and
   at least one memory storing instructions which, when executed by the at least one processor, cause the system to at least perform:

   displaying a graphical user interface (GUI) comprising a two-dimensional (2D) image representative of estimated diameters of an endoluminal tract, the 2D image having a first dimension representative of endoluminal positions, a second dimension representative of points in time, and image pixels having pixel values representative of endoluminal diameter, wherein the endoluminal positions comprise a position of an esophago-gastric junction (EGJ);
   displaying, in the GUI, at least one user interface element among:

   a first user interface element marking location of the EGJ,
   a second user interface element marking time and duration of a filling volume event in which an endoluminal functional lumen imaging probe (EndoFLIP) is marked as being filled with liquid,
   a third user interface element marking time and duration of a dry catheter artifact (DCA) event, and
   a plurality of fourth user interface elements marking pressure peak points of pressure data captured by the EndoFLIP;

   determining usable data from the 2D image based on the at least one user interface element;
   computing, based on the usable data from the 2D image, at least one of: a distensibility index, or a maximum diameter of the EGJ; and
   displaying at least one of: the distensibility index, or the maximum diameter of the EGJ.

2. The system of claim 1, wherein the first user interface element marking the location of the EGJ is movable by a user to mark a new location of the EGJ, and
   wherein the usable data of the 2D image excludes data in the 2D image above and data in the 2D image below the marked location of the EGJ.

3. The system of claim 1 or claim 2, wherein the second user interface element marking the time and duration of a filling volume event is movable by a user to mark a different start time of the filling volume event, is adjustable by the user to mark a different duration of the filling volume event, and is deletable by the user, and
   wherein the usable data of the 2D image excludes data in the 2D image encompassed by the time and duration marked by the second user interface element.

4. The system of any one of claims 1-3, wherein the third user interface element marking the time and duration of a DCA movable by a user to mark a different start time of the DCA event, is adjustable by the user to mark a different duration of the DCA event, and is deletable by the user, and

wherein the usable data of the 2D image excludes data in the 2D image encompassed by the time and duration marked by the third user interface element.

5. The system of any one of claims 1-4, wherein the plurality of fourth user interface elements marking the pressure peak points are movable by a user to mark different pressure peak points and are deletable by a user, and
wherein the usable data of the 2D image excludes data of the 2D image that correspond to pressure points different from the pressure points marked by the plurality of fourth user interface elements.

6. A processor-implemented method comprising:

displaying a graphical user interface (GUI) comprising a two-dimensional (2D) image representative of estimated diameters of an endoluminal tract, the 2D image having a first dimension representative of endoluminal positions, a second dimension representative of points in time, and image pixels having pixel values representative of endoluminal diameter, wherein the endoluminal positions comprise a position of an esophago-gastric junction (EGJ);
displaying, in the GUI, at least one user interface element among:

a first user interface element marking location of the EGJ,
a second user interface element marking time and duration of a filling volume event in which an endoluminal functional lumen imaging probe (EndoFLIP) is marked as being filled with liquid,
a third user interface element marking time and duration of a dry catheter artifact (DCA) event, and
a plurality of fourth user interface elements marking pressure peak points of pressure data captured by the EndoFLIP;

determining usable data from the 2D image based on the at least one user interface element;
computing, based on the usable data from the 2D image, at least one of: a distensibility index, or a maximum diameter of the EGJ; and
displaying at least one of: the distensibility index, or the maximum diameter of the EGJ.

7. The processor-implemented method of claim 6, wherein the first user interface element marking the location of the EGJ is movable by a user to mark a new location of the EGJ, and
wherein the usable data of the 2D image excludes data in the 2D image above and data in the 2D image below the marked location of the EGJ.

8. The processor-implemented method of claim 6 or claim 7, wherein the second user interface element marking the time and duration of a filling volume event is movable by a user to mark a different start time of the filling volume event, is adjustable by the user to mark a different duration of the filling volume event, and is deletable by the user, and
wherein the usable data of the 2D image excludes data in the 2D image encompassed by the time and duration marked by the second user interface element.

9. The processor-implemented method of any one of claims 6-8, wherein the third user interface element marking the time and duration of a DCA movable by a user to mark a different start time of the DCA event, is adjustable by the user to mark a different duration of the DCA event, and is deletable by the user, and
wherein the usable data of the 2D image excludes data in the 2D image encompassed by the time and duration marked by the third user interface element.

10. The processor-implemented method of any one of claims 6-9, wherein the plurality of fourth user interface elements marking the pressure peak points are movable by a user to mark different pressure peak points and are deletable by a user, and
wherein the usable data of the 2D image excludes data of the 2D image that correspond to pressure points different from the pressure points marked by the plurality of fourth user interface elements.

11. A non-transitory processor-readable medium storing instructions which, when executed by at least one processor of a system, cause the system to at least perform:

displaying a graphical user interface (GUI) comprising a two-dimensional (2D) image representative of estimated diameters of an endoluminal tract, the 2D image having a first dimension representative of endoluminal positions, a second dimension representative of points in time, and image pixels having pixel values representative of endoluminal diameter, wherein the endoluminal positions comprise a position of an esophago-gastric junction (EGJ);

displaying, in the GUI, at least one user interface element among:

a first user interface element marking location of the EGJ,

a second user interface element marking time and duration of a filling volume event in which an endoluminal functional lumen imaging probe (EndoFLIP) is marked as being filled with liquid,

a third user interface element marking time and duration of a dry catheter artifact (DCA) event, and

a plurality of fourth user interface elements marking pressure peak points of pressure data captured by the EndoFLIP;

determining usable data from the 2D image based on the at least one user interface element;

computing, based on the usable data from the 2D image, at least one of: a distensibility index, or a maximum diameter of the EGJ; and

displaying at least one of: the distensibility index, or the maximum diameter of the EGJ.

12. The non-transitory processor-readable medium of claim 11, wherein the first user interface element marking the location of the EGJ is movable by a user to mark a new location of the EGJ, and

wherein the usable data of the 2D image excludes data in the 2D image above and data in the 2D image below the marked location of the EGJ.

13. The non-transitory processor-readable medium of claim 11 or claim 12, wherein the second user interface element marking the time and duration of a filling volume event is movable by a user to mark a different start time of the filling volume event, is adjustable by the user to mark a different duration of the filling volume event, and is deletable by the user, and

wherein the usable data of the 2D image excludes data in the 2D image encompassed by the time and duration marked by the second user interface element.

14. The non-transitory processor-readable medium of any one of claims 11-13, wherein the third user interface element marking the time and duration of a DCA movable by a user to mark a different start time of the DCA event, is adjustable by the user to mark a different duration of the DCA event, and is deletable by the user, and

wherein the usable data of the 2D image excludes data in the 2D image encompassed by the time and duration marked by the third user interface element.

15. The non-transitory processor-readable medium of any one of claims 11-14, wherein the plurality of fourth user interface elements marking the pressure peak points are movable by a user to mark different pressure peak points and are deletable by a user, and

wherein the usable data of the 2D image excludes data of the 2D image that correspond to pressure points different from the pressure points marked by the plurality of fourth user interface elements.

**FIG. 1**

EP 4 643 771 A1

200

210 Database

220 Processor

230 Memory

250 GPU

240 Network Interface

**FIG. 2**

FIG. 3

FIG. 4

FIG. 5

FIG. 6

EP 4 643 771 A1

FIG. 7

FIG. 8

FIG. 9

**FIG. 10**

**FIG. 11**

FIG. 12

FIG. 13

1402
Accessing a two-dimensional (2D) image representative of estimated diameters of an endoluminal tract, where the 2D image has a first dimension representative of endoluminal positions, a second dimension representative of points in time, and image pixels having pixel values representative of endoluminal diameter, where the endoluminal positions include a position of an esophago-gastric junction

1404
Determining contours around a pixel subset of the image pixels, where the pixel subset contains pixels which represent endoluminal diameters that exceed a threshold diameter

1406
For the contours, identifying a lowest contour point of each time point of the contours, to provide lowest contour points

1408
Determining local minimum points of the lowest contour points

1410
Estimating a location of the esophago-gastric junction based on the local minimum points

1412
Displaying the estimated location of the esophago-gastric junction relative to the 2D image

FIG. 14

**FIG. 15**

EP 4 643 771 A1

**FIG. 16**

**FIG. 17**

EP 4 643 771 A1

**FIG. 18**

1902 ─┐

Displaying a graphical user interface (GUI) including a two-dimensional (2D) image representative of estimated diameters of an endoluminal tract, where the 2D image has a first dimension representative of endoluminal positions, a second dimension representative of points in time, and image pixels having pixel values representative of endoluminal diameter, where the endoluminal positions include a position of an esophago-gastric junction (EGJ)

1904 ─┐

Displaying, in the GUI, at least one user interface element among: a first user interface element marking location of the EGJ, a second user interface element marking time and duration of a filling volume event in which an endoluminal functional lumen imaging probe (EndoFLIP) is marked as being filled with liquid, a third user interface element marking time and duration of a dry catheter artifact (DCA) event, and a plurality of fourth user interface elements marking pressure peak points of pressure data captured by the EndoFLIP

1906 ─┐

Determining usable data from the 2D image based on the at least one user interface element

1908 ─┐

Computing, based on the usable data from the 2D image, at least one of: a distensibility index, or a maximum diameter of the EGJ

1910 ─┐

Displaying at least one of: the distensibility index, or the maximum diameter of the EGJ

## FIG. 19

Europäisches
Patentamt

European
Patent Office

Office européen
des brevets

# EUROPEAN SEARCH REPORT

**Application Number**

EP 25 17 3707

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/016443 A1 (ZIFAN ALI [US] ET AL) 18 January 2024 (2024-01-18) | 1-5, 11-15 | INV. A61B5/107 |
| Y | * paragraph [0001] - paragraph [0020] * <br> * paragraph [0038] - paragraph [0039] * <br> * paragraph [0043] - paragraph [0051] * <br> * paragraph [0054] - paragraph [0055] * <br> ----- | 1-15 | A61B5/00 |
| Y | US 2023/363695 A1 (ETEMADI MOZZIYAR [US] ET AL) 16 November 2023 (2023-11-16) <br> * paragraph [0003] - paragraph [0006] * <br> * paragraph [0059] * <br> * paragraph [0061] * <br> * paragraph [0103] - paragraph [0104] * <br> ----- | 1-15 | |
| A | US 2014/336527 A1 (PARKS THOMAS R [US]) 13 November 2014 (2014-11-13) <br> * paragraph [0065] - paragraph [0066] * <br> * paragraph [0082] * <br> * paragraph [0086] - paragraph [0096] * <br> * paragraph [0114] - paragraph [0117] * <br> * paragraph [0127] - paragraph [0129] * <br> ----- | 1-15 | |
| A | CARLSON DUSTIN A. ET AL: "Classifying Esophageal Motility by FLIP Panometry: A Study of 722 Subjects With Manometry", AMERICAN JOURNAL OF GASTROENTEROLOGY, vol. 116, no. 12, 20 October 2021 (2021-10-20), pages 2357-2366, XP093313023, US ISSN: 0002-9270, DOI: 10.14309/ajg.0000000000001532 Retrieved from the Internet: URL:https://pmc.ncbi.nlm.nih.gov/articles/ PMC8825704/pdf/nihms-1742756.pdf> [retrieved on 2025-09-09] * the whole document * <br> ----- <br> -/-- | 1-15 | |

**TECHNICAL FIELDS SEARCHED (IPC)**

A61B
G16H

The present search report has been drawn up for all claims

| Place of search | Date of completion of the search | Examiner |
|---|---|---|
| The Hague | 9 September 2025 | Loveniers, Kris |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................................
& : member of the same patent family, corresponding document

page 1 of 2

Europäisches
Patentamt

European
Patent Office

Office européen
des brevets

**EUROPEAN SEARCH REPORT**

Application Number

EP 25 17 3707

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2018/008156 A1 (PANDOLFINO JOHN ERIK [US] ET AL) 11 January 2018 (2018-01-11)<br>* paragraph [0035] *<br>* paragraph [0039] - paragraph [0041] *<br>----- | 1-15 | |
| | | | **TECHNICAL FIELDS SEARCHED (IPC)** |

The present search report has been drawn up for all claims

| Place of search | Date of completion of the search | Examiner |
|---|---|---|
| The Hague | 9 September 2025 | Loveniers, Kris |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
...............................................................................................
& : member of the same patent family, corresponding
   document

EPO FORM 1503 03.82 (P04C01)

page 2 of 2

## ANNEX TO THE EUROPEAN SEARCH REPORT
## ON EUROPEAN PATENT APPLICATION NO.

EP 25 17 3707

This annex lists the patent family members relating to the patent documents cited in the above-mentioned European search report.
The members are as contained in the European Patent Office EDP file on
The European Patent Office is in no way liable for these particulars which are merely given for the purpose of information.

09-09-2025

| Patent document cited in search report | Publication date | Patent family member(s) | Publication date |
|---|---|---|---|
| US 2024016443 A1 | 18-01-2024 | US 2024016443 A1<br>WO 2022094153 A1 | 18-01-2024<br>05-05-2022 |
| US 2023363695 A1 | 16-11-2023 | US 2023363695 A1<br>WO 2022061346 A1 | 16-11-2023<br>24-03-2022 |
| US 2014336527 A1 | 13-11-2014 | US 2006004304 A1<br>US 2014336527 A1 | 05-01-2006<br>13-11-2014 |
| US 2018008156 A1 | 11-01-2018 | CA 2975603 A1<br>US 2018008156 A1<br>WO 2016126701 A1 | 11-08-2016<br>11-01-2018<br>11-08-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63641126

- US 63641132

**Non-patent literature cited in the description**

- **S. SUZUKI et al.** Topological structural analysis of digitized binary images by border following. *Computer Vision, Graphics, and Image Processing*, 1985, vol. 30 (1), 32-46